# EUROPEAN PATENT SPECIFICATION

(11) **EP 2 937 085 B1**
(45) Date of publication and mention of the grant of the patent: **10.07.2019**
(21) Application number: 15164200.6
(22) Date of filing: 21.05.2008
(51) Int. Cl.: A61K 31/445, A61K 31/4709, A61P 25/28

(54) **A MEDICAMENT COMPRISING A CARBOSTYRIL DERIVATIVE AND DONEZEPIL FOR TREATING ALZHEIMER'S DISEASE**
MEDIKAMENTE MIT EINEM CARBOSTYRILDERIVAT UND DONEZEPIL ZUR BEHANDLUNG VON MORBUS ALZHEIMER
MÉDICAMENT COMPRENANT UN DÉRIVÉ DE CARBOSTYRILE ET DONEZEPIL POUR TRAITER LA MALADIE D'ALZHEIMER

(30) Priority: 22.05.2007 JP 2007135367
(43) Date of publication of application: 28.10.2015
(62) Divisional of application: 08764779.8
(73) Proprietor: Otsuka Pharmaceutical Co., Ltd., Tokyo 101-8535 (JP); Juntendo University, Tokyo 113-8421 (JP)
(72) Inventor: Arai, Heii, Tokyo, 113-8421 (JP)
(74) Representative: dompatent von Kreisler Selting Werner - Partnerschaft von Patent- und Rechtsanwälten mbB

(56) References cited:
- WO-A-2004/075897
- US-A1- 2004 229 913
- LEE ET AL: "Concurrent administration of cilostazol with donepezil effectively improves cognitive dysfunction with increased neuroprotection after chronic cerebral hypoperfusion in rats", BRAIN RESEARCH, ELSEVIER, AMSTERDAM, NL, vol. 1185, 28 November 2007 (2007-11-28), pages 246-255, XP022368747, ISSN: 0006-8993

## Description

### Technical Field

The invention relates to a medicament for treating Alzheimer's disease, particularly a combination of a carbostyril derivative of the general formula: 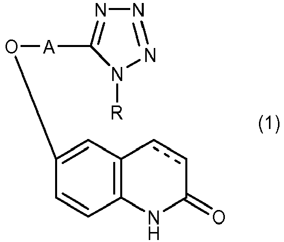
wherein A is a C₁₋₆ alkylene group, R is a cycloalkyl group, the bonding between 3- and 4-positions of the carbostyril skeleton is a single bond or a double bond,
or a salt thereof; and donepezil or a salt thereof, for use in treating Alzheimer's disease.

### Background Art

The carbostyril derivatives of formula (1) or salts thereof and the processes for the preparation thereof are disclosed in JP-63-20235-B and JP-55-35019-A. And it is known that the carbostyril derivatives (1) have platelet aggregation inhibition action, phosphodiesterase (PDE) inhibition action, antiulcer, hypotensive action and antiphlogistic action, and are useful as an antithrombotic agent, a drug for improving cerebral circulation, an antiinflammatory agent, an antiulcer drug, an antihypertensive drug, an antiasthmatic drug, a phosphodiesterase inhibitor, etc. In addition, it is known that the compounds are also useful as a medicament for treating allergic disease (JP-5-320050-A). It is also known that the carbostyril derivatives (1) are a medicament for treating Alzheimer's disease (JP-2006-518732-A).

The number of patients suffering from dementia in Japan was estimated at 1,890,000 people and the prevalence rate thereof was estimated at 7.6% in 2005, and thus the number and the rate have been increasing with the advance of aging of society. It is understood that the number of patients suffering from Alzheimer's disease (AD) occupies more than half of the total number of dementia patients.

The major part of AD is thought to be a sporadic case, but familial AD is thought to be included at about 10% which may develop with autosomal dominant inheritance due to a missense mutation of gene such as amyloid precursor protein gene, presenilin-1 and presenilin-2. Especially, women are more apt to suffer from AD. The most important risk factor of AD is an aging. The prevalence rate of AD increases with age, and thus the majority of AD is late-onset AD which develops on/after 65 years old. AD patients may show various pathologic conditions depending on the disorder of a neurotransmitter such as acetylcholine, the formation of senile plaques through the intracerebral accumulation of amyloid β protein, the intraneuronal accumulation of abnormal filaments comprising phosphorylated tau and other substances, the severe atrophy of a brain through shedding neuronal cells, etc. The core symptoms of AD include memory impairment, aphasia, cognitive impairment, disorder of executive function, and associated symptom; many of which are euphoric. However, some symptoms of AD exhibit adverse conditions such as lack of motivation, depression, bad temper, ill temper from the beginning of the onset.

For the core symptoms of AD, donepezil hydrochloride (commercial name: Aricept) is broadly used in clinical practice (JP-2578475-B; US 2004/229913 A1). For the associated symptom such as insomnia, ill temper, and paranoia, donepezil hydrochloride is also useful as a palliative therapy (Japanese Journal of Psychiatric Treatment, Vol.21, Supplement, Page. 302-305, October 15, 2006, Tsuneyoshi Ohta, Heii Arai). However, the effect of the palliative therapy with donepezil hydrochloride is apt to descend as used in a long term. Thus, donepezil hydrochloride also has a problem that it is hard to continuously and sufficiently suppress the development of the pathologic condition since the medicament is necessary to be administered in a long-term.

### Disclosure of Invention

As mentioned above, donepezil hydrochloride (commercial name: Aricept®) has been widely used as a medicament for treating Alzheimer's disease, however, it has been still desired to develop a more effective medicament for treating Alzheimer's disease which can suppress lowering of the therapeutic effect of donepezil hydrochloride through the long-term administration.

The present inventors have intensively studied a new medicament for treating Alzheimer's disease, and have found that a combination of a carbostyril derivative of the above formula (1), especially 6-[4-(1-cyclohexyl-1H-tetrazol-5-yl)butoxy]-3,4-dihydrocarbostyril (cilostazol) or a salt thereof, and donepezil or a salt thereof exhibits an excellent synergistic action for treating Alzheimer's disease, and then have accomplished the present invention. Especially, the present inventors have found that the combination of the present invention could exhibit an excellent effect improving the action of donepezil hydrochloride which had descended due to long-term administration of donepezil hydrochloride. In addition, the combination or the drug combination of the present invention exhibits fast-acting and low-toxicity, and hence it can be administered over long term. The combination of the present invention is also useful for treating Alzheimer's disease from the viewpoint of safety.

The present invention provides for a combination of a carbostyril derivative of the general formula: 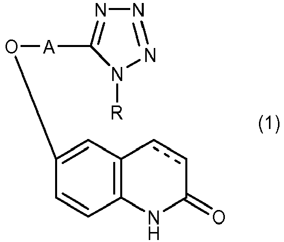
wherein A is a C₁₋₆ alkylene group, R is a cycloalkyl group, the bonding between 3- and 4-positions of the carbostyril skeleton is a single bond or a double bond,
or a salt thereof, and donepezil or a salt thereof, for use in treating Alzheimer's disease.

The present invention also provides for a combination of 6-[4-(1-cyclohexyl-1H-tetrazol-5-yl)butoxy]-3,4-dihydrocarbostyril (cilostazol) or a salt thereof, and donepezil or a salt thereof for use in treating Alzheimer's disease.

The present invention also provides for a combination of the carbostyril derivative (1) and donepezil hydrochloride for use in treating Alzheimer's disease.

The present invention also provides for the use of the carbostyril derivative (1) or a salt thereof, and donepezil or a salt thereof for preparing a medicament for treating Alzheimer's disease.

The present invention also provides for the use of donepezil or a salt thereof in combination with the carbostyril derivative (1) or a salt thereof, for preparing a medicament for treating Alzheimer's disease.

The present invention also provides for the use of the carbostyril derivative (1) or a salt thereof for preparing a medicament for improving the descended action of donepezil or a salt thereof in the treatment of Alzheimer's disease.

Finally, the present invention provides for the carbostyril derivative (1) or a salt thereof for use in improving the descended action of donepezil or a salt thereof in the treatment of Alzheimer's disease due to long-term administration.

According to the present invention, the combination of the carbostyril derivative (1), especially 6-[4-(1-cyclohexyl-1H-tetrazol-5-yl)butoxy]-3,4-dihydrocarbostyril or a salt thereof, and donepezil hydrochloride exhibits effective therapeutic and prophylactic action for Alzheimer's disease.

### Brief Description of Drawings

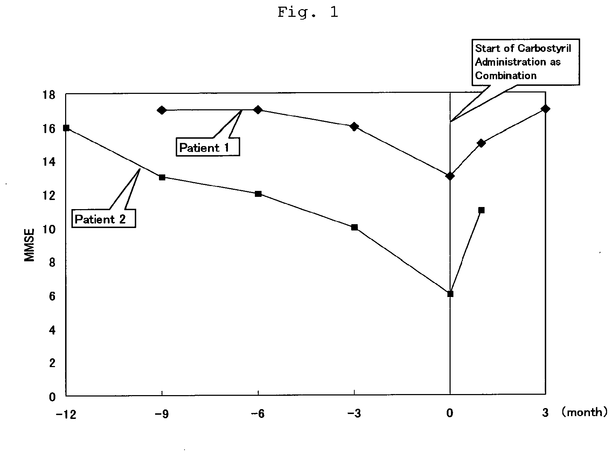

Fig. 1 denotes theoretic effect for Alzheimer's disease using donepezil hydrochloride together with cilostazol as a combination.

### Best Mode for Carrying Out the Invention

The carbostyril derivative which is used in combination with donepezil or a salt thereof is a tetrazolylalkoxy-dihydrocarbostyril derivative of the formula: 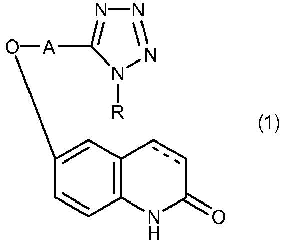
wherein A is a C₁₋₆ alkylene group, R is a cycloalkyl group, the bonding between 3- and 4-positions of the carbostyril skeleton is a single bond or a double bond,
or a salt thereof.

In the above formula (1), the cycloalkyl group includes C₃-C₈ cycloalkyl groups such as cyclopropyl, cyclobutyl, cyclopentyl, cyclohexyl, cycloheptyl, and cyclooctyl. Preferred cycloalkyl group is cyclohexyl. The C₁₋₆ alkylene group includes methylene, ethylene, propylene, tetramethylene, butylene, and pentylene, among which tetramethylene is preferred.

The preferred carbostyril derivative is 6-[4-(1-cyclohexyl-1H-tetrazol-5-yl)butoxy]-3,4-dihydrocarbostyril, which has been put on the market in the trade name of cilostazol as an antiplatelet agent.

The carbostyril derivative (1) can be easily converted to a salt thereof by getting it treated with a pharmaceutically acceptable acid. The acid includes, for example, inorganic acids such as hydrochloric acid, sulfuric acid, phosphoric acid, and hydrobromic acid; and organic acids such as oxalic acid, maleic acid, fumaric acid, malic acid, tartaric acid, citric acid, and benzoic acid.

These carbostyril derivatives (1) and salts thereof and processes for preparation thereof are disclosed in JP-55-35019-A (relevant to U.S. Patent 4,277,479).

The other active ingredient is donepezil whose chemical name is 1-benzyl-4-[(5,6-dimethoxyindan-1-one)-2-yl]methylpiperazine. A hydrochloride thereof has been put on the market as a medicament for treating Alzheimer's disease (donepezil hydrochloride, commercial name: Aricept®). This compound is disclosed in JP-2578475-B. 1-Benzyl-4-[(5,6-dimethoxyindan-1-one)-2-yl]methylpiperazine can be easily transformed to a salt form thereof using a pharmaceutical acceptable acid.

The acid includes, for example, inorganic acids such as hydrochloric acid, sulfuric acid, phosphoric acid and hydrobromic acid, and organic acids such as oxalic acid, maleic acid, fumaric acid, malic acid, tartaric acid, citric acid and benzoic acid. Amongst them, a hydrochloride thereof, donepezil hydrochloride is preferred.

These active ingredients, the carbostyril derivative (1) or a salt thereof and donepezil or a salt thereof may be administered together or separately, at the same time or different time. These ingredients may usually be used in a conventional pharmaceutical formulation. Then, these ingredients may be prepared in a single dosage form or in separate dosage forms.

The dose of these active ingredients is not limited to a specific range. The carbostyril derivatives (1) or a salt thereof may be used in an amount of 50 to 200 mg/day per an adult (50 kg of body weight), which is administered once a day or two to several times per day. Donepezil may be used in an amount of 0.1 to 300 mg/day, preferably 1 to 10 mg/day per an adult (50 kg of body weight), which is usually administered one to four times per day. When these ingredients are prepared in a single dosage form, they are incorporated in a ratio of 0.025 to 1.0 parts by weight of donepezil per 1 part by weight of the carbostyril derivative (1) or a salt thereof. And, the drug combination may include the sum of the ingredients in 0.1 - 70 % (w/w) per the preparation, but not limited thereto.

The each dosage form used for the combination of the present invention includes, for example, the dosage forms exemplified in JP-10-175864-A, and typically an oral solid dosage form such as tablets and capsules, an oral liquid dosage form such as syrups and elixirs, a parenteral dosage form such as injections, and
an inhalant.

The preparations, such as tablets, capsules and liquid for oral administration, may be prepared by a conventional method. The tablets may be prepared by mixing the active ingredient(s) with conventional pharmaceutical carriers such as gelatin, starches, lactose, magnesium stearate, talc and gum arabic. The capsules may be prepared by mixing the active ingredient(s) with inert pharmaceutical fillers or diluents and filling hard gelatin capsules or soft capsules with the mixture. The oral liquid preparations such as syrups or elixirs are prepared by mixing the active ingredient(s) with sweetening agents (e.g. sucrose), preservatives (e.g. methylparaben, propylparaben), colorants and flavors. The preparations for parenteral administration may also be prepared by a conventional method, for example, by dissolving the active ingredient(s) in a sterilized aqueous carrier, preferably water or a saline solution. The preferred liquid preparation suitable for parenteral administration is prepared by dissolving the daily dose of the active ingredients as mentioned above in water and an organic solvent and further in a polyethylene glycol having a molecular weight of 300 to 5000, in which preferably a lubricant such as sodium carboxymethylcellulose, methylcellulose, polyvinylpyrrolidone, and polyvinyl alcohol is incorporated. Preferably, the above liquid preparations may further comprise a disinfectant (e.g. benzyl alcohol, phenol, thimerosal), a fungicide, and further optionally an isotonic agent (e.g. sucrose, sodium chloride), a topical anesthetic, a stabilizer and a buffer. In view of keeping stability, the preparation for parenteral administration may be put in capsules, followed by removing the aqueous medium by a conventional lyophilizing technique. The preparation can be recovered into a liquid preparation by dissolving in an aqueous medium when used. The inhalants may be prepared by a conventional method. That is, the inhalants may be prepared by getting an active compound to a powder or liquid state, mixing it into propellants and/or carriers for inhalant, and charging an appropriate vaporizer with the mixture. Ordinarily, a mechanical powder vaporizer can be used when the active compound is a powder, and a vaporizer such as a nebulizer can be used when the compound is a liquid. In addition, the inhalant may optionally comprise a surfactant, an oil, a flavor, a cyclodextrin or a derivative thereof which has been used when necessary.

The examples of the above-mentioned additive agents, processes thereof, or other things include what JP-10-175864-A discloses.

### Example

To two women suffering from Alzheimer's disease (one was 63 years old and the other was 52 years old), donepezil hydrochloride (Aricept®) had been administered in a dose of 5 mg/day for 12 months and 9 months, respectively. During under the administration, the mini-mental state examination (MMSE) was carried out for the women (Journal of psychiatric research. 1975 Nov; 12 (3): 189-98), and the result was shown in Table 1 and Figure 1. At the beginning time of the experiment (0 month), the administration of cilostazol in a dose of 100 mg/day started as the combination with donepezil hydrochloride. At that time, the MMSE score which had been descending until that time was quickly enhanced. According to the above result, it has found that the administration of cilostazol as the combination with donepezil hydrochloride can recover the effect of donepezil hydrochloride which tended to descend due to long-term administration of donepezil hydrochloride. And it has also found that the combination of donepezil hydrochloride and cilostazol can provide a potent therapeutic effect for dementia such as Alzheimer's disease.

**Table 1**

| Patient No. | Age | Sex | MMSE Score | | | | | | |
|---|---|---|---|---|---|---|---|---|---|
| | | | -12 Ms | -9 Ms | -6 Ms | -3 Ms | 0 M | 1 M | 3 Ms |
| 1 | 63 | F | - | 17 | 17 | 16 | 13 | 15 | 17 |
| 2 | 52 | F | 16 | 13 | 12 | 10 | 6 | 11 | - |

| | | | | | | | | | |
|---|---|---|---|---|---|---|---|---|---|
| M(s) denotes "month(s)". The administration of cilostazol in the combination started at 0 M. | | | | | | | | | |

## Claims

1. A combination of a carbostyril derivative of the general formula: 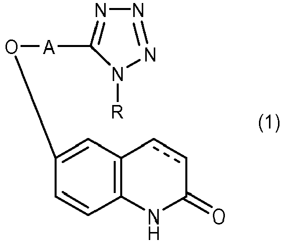
wherein A is a C₁₋₆ alkylene group, R is a cycloalkyl group, the bonding between 3- and 4-positions of the carbostyril skeleton is a single bond or a double bond, or a salt thereof; and donepezil or a salt thereof,
for use in treating Alzheimer's disease.

2. The combination for use according to claim 1, wherein the carbostyril derivative is 6-[4-(1-cyclohexyl-1H-tetrazol-5-yl)butoxy]-3,4-dihydrocarbostyril or a salt thereof.

3. The combination for use according to claim 1 or 2, wherein the salt of donepezil is donepezil hydrochloride.

4. Use of the carbostyril derivative or a salt thereof as set forth in claim 1 or 2 in combination with donepezil or a salt thereof for preparing a medicament for treating Alzheimer's disease.

5. Use of donepezil or a salt thereof in combination with the carbostyril derivative or a salt thereof as set forth in claim 1 or 2 for preparing a medicament for treating Alzheimer's disease.

6. The use of claim 4 or 5, wherein the salt of donepezil is donepezil hydrochloride.

7. Use of the carbostyril derivative or a salt thereof as set forth in claim 1 or 2 for preparing a medicament for improving the descended action of donepezil or a salt thereof in the treatment of Alzheimer's disease.

8. The use of claim 7, wherein the salt of donepezil is donepezil hydrochloride.

9. The carbostyril derivative or a salt thereof as set forth in claim 1 or 2 for use in improving the descended action of donepezil or a salt thereof in the treatment of Alzheimer's disease due to long-term administration.

10. The carbostyril derivative or a salt thereof for use in improving the descended action of donepezil or a salt thereof of claim 9, wherein the salt of donepezil is donepezil hydrochloride.

## Patentansprüche

1. Kombination aus einem Carbostyrilderivat der allgemeinen Formel 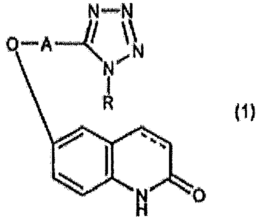 wobei A eine C₁₋₆-Alkylengruppe ist, R eine Cycloalkylgruppe ist, die Bindung zwischen den Positionen 3 und 4 des Carbostyrilgerüsts eine Einfachbindung oder eine Doppelbindung ist, oder einem Salz davon und Donepezil oder einem Salz davon zur Verwendung bei der Behandlung der Alzheimer-Krankheit.

2. Kombination zur Verwendung gemäß Anspruch 1, wobei es sich bei dem Carbostyrilderivat um 6-[4-(1-Cyclohexyl-1H-tetrazol-5-yl)butoxy]-3,4-dihydrocarbostyril oder ein Salz davon handelt.

3. Kombination zur Verwendung gemäß Anspruch 1 oder 2, wobei es sich bei dem Salz von Donepezil um Donepezil-Hydrochlorid handelt.

4. Verwendung des Carbostyrilderivats oder seines Salzes gemäß Anspruch 1 oder 2 in Kombination mit Donepezil oder seines Salzes für die Herstellung eines Medikaments zur Behandlung der Alzheimer-Krankheit.

5. Verwendung von Donepezil oder eines Salzes davon in Kombination mit dem Carbostyrilderivat oder seinem Salz gemäß Anspruch 1 oder 2 für die Herstellung eines Medikaments zur Behandlung der Alzheimer-Krankheit.

6. Verwendung gemäß Anspruch 4 oder 5, wobei es sich bei dem Salz von Donepezil um Donepezil-Hydrochlorid handelt.

7. Verwendung des Carbostyrilderivats oder seines Salzes gemäß Anspruch 1 oder 2 für die Herstellung eines Medikaments zur Verbesserung der gesunkenen Wirkung von Donepezil oder eines Salzes davon bei der Behandlung der Alzheimer-Krankheit.

8. Verwendung gemäß Anspruch 7, wobei es sich bei dem Salz von Donepezil um Donepezil-Hydrochlorid handelt.

9. Carbostyrilderivat oder sein Salz gemäß Anspruch 1 oder 2 zur Verwendung bei der Verbesserung der aufgrund einer langfristigen Verabreichung gesunkenen Wirkung von Donepezil oder eines Salzes davon bei der Behandlung der Alzheimer-Krankheit.

10. Carbostyrilderivat oder ein Salz davon zur Verwendung bei der Verbesserung der gesunkenen Wirkung von Donepezil oder eines Salzes davon gemäß Anspruch 9, wobei es sich bei dem Salz von Donepezil um Donepezil-Hydrochlorid handelt.

## Revendications

1. Combinaison d'un dérivé de carbostyrile de la formule générale : 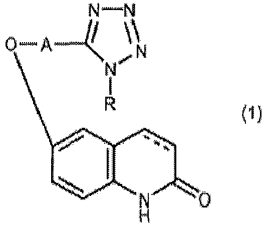
dans laquelle A est un groupe alkylène en C₁₋₆, R est un groupe cycloalkyle, la liaison entre les positions 3 et 4 du squelette carbostyrile est une liaison simple ou une liaison double, ou d'un sel de celui-ci ; et de donépézil ou d'un sel de celui-ci,
pour une utilisation dans le traitement de la maladie d'Alzheimer.

2. Combinaison pour une utilisation selon la revendication 1, dans laquelle le dérivé de carbostyrile est le 6-[4-(1-cyclohexyl-1H-tétrazol-5-yl)butoxy]-3,4-dihydrocarbostyrile ou un sel de celui-ci.

3. Combinaison pour une utilisation selon la revendication 1 ou 2, dans laquelle le sel de donépézil est l'hydrochlorure de donépézil.

4. Utilisation du dérivé de carbostyrile ou d'un sel de celui-ci selon la revendication 1 ou 2 en combinaison avec du donépézil ou un sel de celui-ci pour la préparation d'un médicament pour le traitement de la maladie d'Alzheimer.

5. Utilisation de donépézil ou d'un sel de celui-ci en combinaison avec le dérivé de carbostyrile ou un sel de celui-ci selon la revendication 1 ou 2 pour la préparation d'un médicament pour le traitement de la maladie d'Alzheimer.

6. Utilisation selon la revendication 4 ou 5, dans laquelle le sel de donépézil est l'hydrochlorure de donépézil.

7. Utilisation du dérivé de carbostyrile ou d'un sel de celui-ci selon la revendication 1 ou 2 pour la préparation d'un médicament pour améliorer l'action décroissante du donépézil ou d'un sel de celui-ci dans le traitement de la maladie d'Alzheimer.

8. Utilisation selon la revendication 7, dans lequel le sel de donépézil est l'hydrochlorure de donépézil.

9. Dérivé de carbostyrile ou sel de celui-ci selon la revendication 1 ou 2 pour une utilisation dans l'amélioration de l'action décroissante de donépézil ou d'un sel de celui-ci dans le traitement de la maladie d'Alzheimer due à une administration à long terme.

10. Dérivé de carbostyrile ou d'un sel de celui-ci pour une utilisation dans l'amélioration de l'action décroissante de donépézil ou d'un sel de celui-ci selon la revendication 9, dans lequel le sel de donépézil est l'hydrochlorure de donépézil.
